# EUROPÄISCHE PATENTANMELDUNG

(11) **EP 0 687 474 A1**
(43) Veröffentlichungstag der Anmeldung: **20.12.1995**
(21) Anmeldenummer: 95109001.8
(22) Anmeldetag: 12.06.1995
(51) Int. Cl.: A61M 1/28

(54) **Peritonealdialysegerät**

(30) Priorität: 16.06.1994 DE 4421126
(71) Anmelder: Fresenius AG, D-61350 Bad Homburg (DE)
(72) Erfinder: Simon, Wolfgang, Dr., D-97421 Schweinfurt (DE); Pototzky, Günther, D-97453 Schonungen (DE); Groos, Rolf, D-61381 Friedrichsdorf (DE); Schmidt, Günter, D-45721 Haltern (DE)
(74) Vertreter: Fuchs, Luderschmidt & Partner Patentanwälte

(57) **Zusammenfassung**

Peritonealdialysegerät (10) mit einer Bilanzkammer (28), die durch eine Membran (30) in zwei Bilanzkammerhälften (32 und 34) geteilt ist, mit einer Zuführleitungsanordnung (16,20,22,24), einer Abflußanordnung (22,24,20.62), einer Katheterleitungsanordnung (36,38,42), einer Ventilanordnung (46-52), einer Pumpanordnung (60,108,110 und einer Steuereinheit (82), die die Bilanzkammerhälften (32 bzw. 34) mittels der Ventilanordnung (46-52) jeweils in den Füll- bzw. Entleerungsbetrieb schaltet.

Das Peritonealdialysegerät (10,106) arbeitet unter Drucküberwachung in der Füllphase volumetrisch und in der Entleerungsphase druckgesteuert und ermittelt jeweils aus der Anzahl der Bilanzkammerhübe, das zu- bzw. abgeförderte Dialysierflüssigkeitsvolumen und aus der Differenz hiervon das Ultrafiltrationsvolumen.

## Beschreibung

Die Erfindung betrifft ein Peritonealdialysegerät mit einer Bilanzieranordnung mit zwei Kammern, die durch eine Ventilanordnung jeweils in eine Füll- bzw. Entleerungsphase umschaltbar sind, einer mit der Bilanzieranordnung über eine Zuführleitung verbundene Peritonealdialysierflüssigkeitsquelle, eine mit einem Peritonealkatheter verbindbaren, von der Bilanzieranordnung abgehende Katheterleitung, eine von der Bilanzieranordnung abgehende Abflußleitung, einer Pumpanordnung zur Förderung von frischer bzw. verbrauchter Peritonealdialysieflüssigkeit und einer Steuereinheit zum Steuern der Ventilanordnung und der Pumpanordnung.

Vorrichtungen zur automatisierten Peritonealdialyse, nachstehend Cycler genannt, haben die Aufgabe, Peritonealdialysierflüssigkeit in definierter Menge und Temperatur in den Bauchraum eines Patienten mittels eines Verweilkatheters zu befördern und nach einer Einwirkzeit wieder zu entfernen, wobei das im Peritonealraum während der Dialysebehandlung erzeugte Ultrafiltrat zugleich mit abgezogen werden soll. Dieser Grundprozeß wiederholt sich in cyclischen Abständen entsprechend den jeweiligen klinischen Verfahren und den individuellen Erfordernissen der Behandlung.

Die Flüssigkeit soll in möglichst kurzer Zeit in vorbestimmter Menge verabreicht und nach der Wirkzeit wiederum in möglichst kurzer Zeit entzogen werden, wobei die entzogene Menge genauer bestimmt werden muß, damit eine hinreichend genaue Aussage über die Bilanz und damit auch die Menge des körpereigenen erzeugten Ultrafiltrats gewährleistet ist. Während des Ein- und Auslaufs der Flüssigkeit darf keine Patientengefährdung oder Befindlichkeitsbeeinträchtigung entstehen. Es ist auch exakt festzustellen, wann der Bauchraum hinreichend leer ist, um den nächsten Einlauf wieder zu starten.

Üblicherweise schließt sich der Patient selbst an einen solchen Cycler bei der Heimdialyse an. Die eigentliche Behandlung erfolgt dann teilweise während des Schlafs, so daß eine einfache Bedienbarkeit und absolute Sicherheit unabdingbar sind. Auch darf die Funktion nicht beeinträchtigt werden durch korposkulare Bestandteile der auslaufenden Flüssigkeit, die besonders bei Entzündungen im Patienten entstehen können. Schließlich muß der Flüssigkeitstransfer unter absolut sterilen Bedingungen ablaufen, da ansonsten die Gefahr der Entstehung einer zum Teil lebensbedrohlichen Peritonitis auftreten kann.

Es sind bereits eine Vielzahl von zum Teil vollautomatischen Peritonealdialysegeräten bekannt, die jedoch die vorstehend genannten Anforderungen nur zum Teil erfüllen.

Aus der Münchener Medizinischen Wochenzeitschrift (1972) S. 313 ist ein Peritonealdialysegerät bekannt, bei dem volumetrisch eine bestimmte Menge Peritonealdialysierflüssigkeit einem Patienten zugeführt wird. Nach der Dialysephase wird diese Flüssigkeit dem Patienten wieder entzogen, wobei eine Volumenbilanz erstellt werden soll. Es ist jedoch nicht ersichtlich, wie die Ultrafiltrationsmenge bestimmt werden soll.

Aus dem US-Patent 3 709 222 ist ebenfalls ein automatisches Peritonealdialysegerät bekannt, das mit Hilfe von Proportionierungskammern in zeitabhängiger Weise Dialysierflüssigkeit einem Patienten zuführt. Eine volumetrische Kontrolle der geförderten Flüssigkeiten ist mit dieser Anordnung nicht zu erreichen, da dort expandierbare Kammern, beispielsweise die Rücklaufkammer in Verbindung mit wegabhängigen Sensoren, eingesetzt werden, so daß keine genaue volumetrische Kontrolle gegeben ist.

Weitere Peritonealdialysegeräte sind in den US-Patenten 4 096 859, 4 412 917, 4 381 003, und 5 004 459 beschrieben. Bei den Geräten gemäß der beiden zuerst genannten US-Patentschriften werden Wägevorrichtungen eingesetzt, die schwierig zu handhaben sind und daher für einen möglichst einfach zu gestaltenden Cycler bereits aus Handhabungsgründen nicht in Frage kommen. Auch die Vorrichtungen gemäß den anderen US-Patenten sind mit Mängeln behaftet, da diese peristaltische Pumpen einsetzen, deren Förderraten bekanntlich vom Eingangsdruck abhängen. Da diese Eingangsdrücke in Abhängigkeit von der Förderart stark variieren, sind solche Pumpanordnungen mit erheblichen volumetrischen Fehlern behaftet, so daß ein Einsatz bei der Ultrafiltrationskontrolle in der Peritonealdialyse hiermit nicht verwirklicht werden kann.

Schließlich beschreibt die EP-A 149 001 ein Peritonealdialysegerät, bei dem die Dialysierflüssigkeit in einem extrakorporalen Kreislauf durch die semipermeable Membran eines Filters hinweg dem Katheter des Patienten zugeführt wird. Obwohl diese Vorrichtung vollautomatisch teils gesteuert, teils geregelt arbeitet, ist diese zu aufwendig und damit auch schwierig zu betreiben, ganz abgesehen davon, daß die dort eingesetzten Disposables zu teuer sind. Das gleiche gilt bei den aus dem DE-G 87 14 464 bekannten Disposable.

Schließlich sind in der Hämodialyse exakt bilanzierende Systeme bekannt, bei denen frische und verbrauchte Dialysierflüssigkeit gegeneinander im geschlossenen Kreislauf in einer Bilanzkammer stetig bilanziert werden.

Der Erfindung liegt daher die Aufgabe zugrunde, ein Peritonealdialysegerät der eingangs erwähnten Art zur Verfügung zu stellen, das in einfacher und zuverlässiger Weise eine genaue Volumenbestimmung der zu- und abgeführten Peritonealdialysierflüssigkeiten ermöglicht.

Die Aufgabe wird dadurch gelöst, daß das Peritonealdialysegerät eine einzige Bilanzkammer aufweist, die durch eine bewegliche, flüssigkeitsundurchlässige Membran in zwei Bilanzkammerhälften getrennt ist, wobei die Bilanzkammerhälften wechselweise über die Ventilanordnung einerseits mit der Quelle für die Peritonealdialysierflüssigkeit im Zuförderbetrieb und mit der Abflußleitung im Abförderbetrieb und andererseits mit der Katheterleitung in Strömungsverbindung sind, ein erster Drucksensor an der Katheterleitung angeordnet ist, auf dessen druckabhängiges Signal die Steuereinheit bei Erreichen eines vorgegebenen Druckwerts die Pumpe inaktiviert, und eine Einrichtung zum Ermitteln der vollständigen Füllung der Bilanzkammer vorgesehen ist, auf deren Signal hin die Steuereinheit die Ventilanordnung jeweils zyklisch umschaltet.

Das erfindungsgemäße Peritonealdialysegerät verfügt über eine Bilanzkammer, die durch eine bewegliche, flüssigkeitsundurchlässige Wand in zwei Hälften geteilt ist. Dabei verdrängt die in einer Hälfte zugeführte Flüssigkeitsmenge durch Verdrängung der Wand die in der anderen Hälfte vorhandene Flüssigkeitsmenge in exakt volumetrischer Weise. Infolgedessen kann mit hoher Genauigkeit das ein- oder auslaufende Volumen bis auf ein Kammervolumen genau (ca. 1% Fehler) bestimmt werden, so daß auch die ultrafiltrierte Menge exakt ermittelt werden kann. Dies hat eine bessere Kontrolle der Behandlung zur Folge bzw. die Dialysebehandlung kann hierdurch optimiert werden. Im übrigen wird eine Patientenschädigung durch Überfüllung mittels Druckkontrolle sicher vermieden.

Das Füllen und Entleeren läßt sich in physiologisch kürzester Zeit dadurch durchführen, daß der Ein- und Auslaufdruck gesteuert überwacht wird. So läßt sich beispielsweise ein relativer Über- oder Unterdruck von 100 mbar ohne Probleme überwachen.

Infolgedessen läßt sich eine definierte Behandlung weitgehend unabhängig von der Lage und von der Bewegung des Patienten durchführen, so daß ihm dadurch Restriktionen erspart werden. Die Genauigkeit und Zuverlässigkeit des Systems ist unabhängig von Verschmutzungen (Blut etc.).

Ein besonderer Vorteil der Erfindung ist die Tatsache, daß die Bilanzkammer einschließlich sämtlicher Schlauchzuleitungen als geschlossenes, unbelüftetes Einmal-Gebrauchssystem mit hoher Sterilitätssicherheit ausgebildet ist, wobei sämtliche Teile vorteilhafterweise aus polymeren Materialien bestehen. Eine solche Bilanzkammer ist beispielsweise in der DE-A 41 16 178 beschrieben, die jedoch bei der Hämofiltration eingesetzt wird.

Es ist weiterhin vorteilhaft, wenn dieses Einmal-Gebrauchssystem in Form einer kompakten, einfach handhabbaren Kassette vorliegt, die auf einem entsprechend ausgebildeten Basisgerät jeweils vor der Behandlung anzuordnen ist. Dieses Basisgerät enhält dann die weiteren Betriebsteile, wie Pumpen, Schlauchventile, Drucksensoren und dergleichen.

Es zeigen
- Fig. 1: eine erste Ausführungsform eines Peritonealdialysegeräts in schematischer Darstellung und
- Fig. 2: eine zweite Ausführungsform eines Peritonealdialysegeräts ebenfalls in schematischer Darstellung.

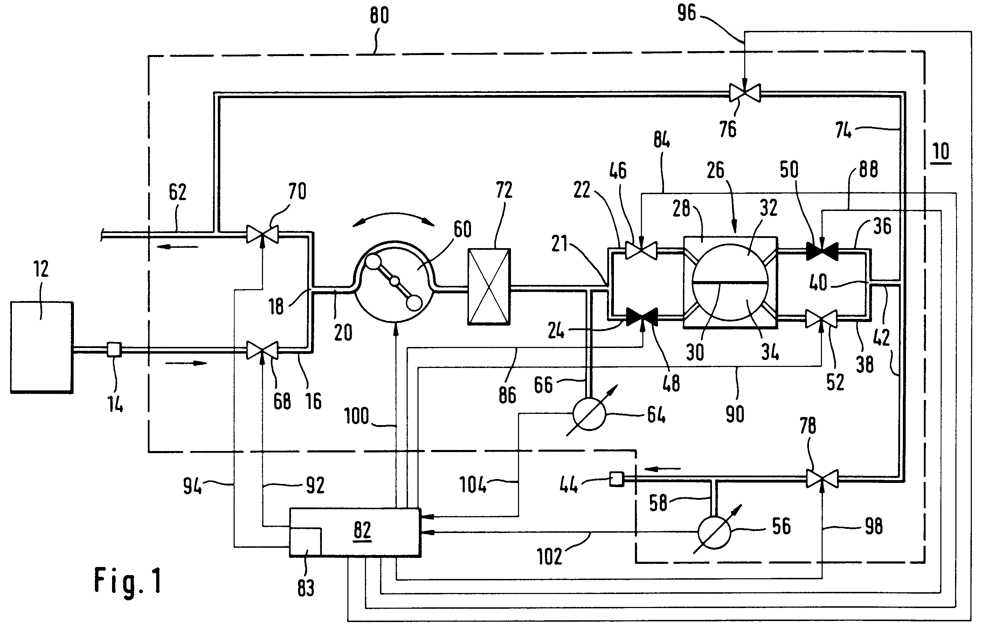

In Fig. 1 ist ein Peritonealdialysegerät 10 in schematischer Darstellung gezeigt. Dieses Peritonealdialysegerät 10 weist eine Quelle 12 für Peritonealdialysierflüssigkeit auf, die üblicherweise aus einer Mehrzahl von Kunststollbeuteln besteht, die mit einer sterilen Peritonealdialysierflüssigkeit gefüllt sind. Diese Beutel sind über eine Konnektoranordnung 14 steril mit mindestens einer Einlaufleitung 16 verbunden. Andererseits kann jedoch aber auch die Peritonealdialysierflüssigkeit on-line aus Elektrolytkonzentraten und einer osmotisch wirksamen Substanz (z.B. Glucose oder Glucosepolymere) durch Vermischen mit Wasser hergestellt werden. Eine derartige Anlage ist ebenfalls unter der Quelle 12 zu verstehen.

Die Eingangsleitung 16 erstreckt sich von der Quelle 12 bzw. der Konnektoranordnung 14 bis zu einem Verzweigungspunkt 18 und geht dort in eine Förderleitung 20 über, die sich an ihrem anderen Ende 21 in eine erste Bilanzkammerleitung 22 und in eine zweite Bilanzkammerleitung 24 verzweigt.

Beide Leitungen 22 und 24 münden jeweils in eine Bilanzkammer 26.

Die Bilanzkammer 26 besteht im wesentlichen aus einem starren Bilanzkammergehäuse 28, die durch eine bewegliche, flüssigkeitsundurchlässige Membran 30 in eine erste Bilanzkammerhälfte 32 und in eine zweite Bilanzkammerhälfte 34 unterteilt ist. Beide Bilanzkammerhälften 32 und 34 bilden dabei die eigentliche Bilanzkammer 26, die ein vorgegebenes konstantes Volumen (vorteilhafterweise etwa 20-30 ml) aufweist.

Wie aus Fig. 1 ersichtlich ist, mündet die erste Bilanzkammerleitung 22 in die erste Bilanzkammerhälfte 32, während die zweite Bilanzkammerleitung 24 in die zweite Bilanzkammerhälfte 34 mündet.

Von der ersten Bilanzkammerhälfte 32 geht eine dritte Bilanzkammerleitung 36 und von der zweiten Bilankammerhälfte 34 geht eine vierte Bilanzkammerleitung 38 ab, die gemeinsam in einem Verzweigungspunkt 40 münden.

Vom Verzweigungspunkt 40 erstreckt sich eine Katheterleitung 42, deren Ende über eine weitere Konnektoranordnung 44 mit einem Peritonealdialysekatheter (nicht gezeigt) verbunden werden kann.

In der ersten, zweiten, dritten bzw. vierten Bilanzkammerleitung 22, 24, 36 bzw. 38 sind jeweils erste, zweite, dritte bzw. vierte Ventile vorgesehen, wobei das erste und das vierte Ventil 46, 52 ein erstes Ventilpaar und die in der Zeichnung dunkel dargestellten zweiten und dritten Ventile 48, 50 ein zweites Ventilpaar darstellen. Erfindungsgemäß werden dabei die ersten und zweiten Ventilpaare gegenläufig aktiviert, d.h. während das erste Ventilpaar geschlossen ist, bleibt das zweite Ventilpaar geöffnet und umgekehrt.

Weiterhin ist die Katheterleitung 42 mit einem Druckdetektor 56 verbunden, und zwar entweder direkt am Leitungsumfang oder aber mittels einer Verbindungsleitung 58, die unter sterilen Bedingungen mit dem Druckdetektor 56 verbunden ist. Dieser Druckdetektor 56 ist in der Lage, Druckunterschiede von 0-200 mbar (Unter- wie Überdrücke) exakt zu bestimmen.

Gemäß der in Fig. 1 gezeigten Ausführungsform ist nur eine Pumpe 60 zur Zu- und Abförderung der Dialysierflüssigkeiten vorgesehen. Diese Pumpe 60 ist in die Förderleitung 20 eingeschaltet.

Wie aus Fig. 1 ersichtlich ist, erfolgt im Uhrzeigersinn eine Förderung von frischer Dialysierflüssigkeit über die Zuleitung 16 in die Förderleitung 20, während gegen den Uhrzeigersinn die Abförderung gebrauchter Dialysierflüssigkeit aus der Förderleitung 20 in eine Abflußleitung 62 erfolgt, die vom Verzweigungspunkt 18 abgeht und in einen nicht gezeigten Abfluß mündet.

Zwischen Bilanzkammer 26 und der Pumpe 60 ist gemäß einer ersten Ausführungsform im Bereich der Förderleitung 20 ein zweiter Druckdetektor 64 als Einrichtung zur Ermittlung der vollständigen Füllung der Bilanzkammer 26 vorgesehen, der entweder unmittelbar mit der Förderleitung 20 verbunden ist oder aber über eine Verbindungsleitung 66 in Druckverbindung ist. Bekanntlich steigt der Druck steil mit Erreichen der Füllung der Bilanzkammer an, so daß der Druckanstieg zum Umschalten der jeweiligen Ventilpaare 46,52;48,50 ggf. in Verbindung mit einem Stillsetzen der Pumpe herangezogen werden kann.

Die in Fig. 1 gezeigte erste Ausführungsform eines Peritonealdialysegerätes 10, die nur über eine Pumpe 60 verfügt, weist vorteilhafterweise in der Einlaufleitung 16 ein fünftes Ventil 68 und in der Abflußleitung 62 ein sechstes Ventil 70 auf, die ebenfalls gegensinnig betätigt werden.

Des weiteren ist eine Heizeinrichtung 72 in Form eines Heizungsbeutels vorgesehen, der, wie in Fig. 1 dargestellt ist, vorteilhafterweise zwischen der Pumpe 70 und der Bilanzkammer 26 in der Förderleitung 20 angeordnet ist. Derartige Heizungsbeutel sind bekannt und dienen zum Aufwärmen der Peritonealdialysierflüssigkeit auf die Körpertemperatur des Patienten. Andererseits kann jedoch aber auch ein solcher Heizungsbeutel 72 in der Zulaufleitung 16 stromauf des fünften Ventils 68 angeordnet sein.

Zu Entlüftungszwecken bzw. zum Druckausgleich geht von der Katheterleitung 42 eine Entlüftungsleitung 74 ab, die in die Abflußleitung 62 stromab des sechsten Ventils 70 mündet. In die Entlüfüngsleitung 74 ist ein siebtes Ventil 76 eingeschaltet.

Schließlich kann in der Kathetherleitung 42 stromauf des ersten Drucksensors 56 und stromab des Abzweigpunktes der Entlüftungsleitung 74 ein achtes Ventil 78 vorgesehen sein.

Sämtliche in Fig. 1 gezeigten Leitungen, die mit der Bilanzkammer 26 verbunden sind, bestehen wie die Bilanzkammer 26 selbst und der Heizbeutel 72 aus polymeren Materialien. Sämtliche Komponenten sind gemäß einer weiteren Ausführungsform in vorbestimmter Lage in einer üblicherweise ebenfalls aus Kunststoff bestehenden Kassette 80 angeordnet, die in Fig. 1 gestrichelt dargestellt ist. Die Ventile wiederum sind vorteilhafterweise als elektrisch aktivierbare Klemmen ausgebildet, die auf die elastische Schlauchwand klemmend einwirken, sobald die Kassette 80 eingelegt ist. Auch die Drucksensoren 56 und 64 wirken vorteilhafterweise berührend auf die Schlauchwand ein und bestimmen den Schlauchinnendruck durch die Verformung des Schlauchs. Andererseits sind jedoch aber auch in den Schlauch integrierte Einmalsensoren einsetzbar.

Zur Steuerung des Peritonealdialysegerätes 10 ist eine Steuereinheit 82 vorgesehen, die über die Steuerleitungen 84-98 mit den ersten bis achten Ventilen 46-52, 68, 70, 76 bzw. 78 verbunden sind. Über eine neunte Steuerleitung 100 ist die Steuereinrichtung 82 mit der Pumpe 60 verbunden. Sie empfängt Signale von dem ersten bzw. zweiten Drucksensor 56 bzw. 64 über die Signalleitungen 102 bzw 104.

Das in Fig. 1 gezeigte Peritonealdialysegerät 10 wird folgendermaßen betrieben:
In der Quelle 12 wird zunächst frische Peritonealdialysierflüssigkeit bereitgestellt. Der Patient wird über den Peritonealdialysekatheter an die Konnektionseinrichtung 44 angeschlossen. Danach wird das Peritonealdialysegerät 10 aktiviert und auf Einlauf gestellt. Hiernach wird das Ventil 68 in der Einlaufleitung 60 geöffnet, während das Ventil 70 in der Auslaufleitung 62 geschlossen bleibt. Die Pumpe 60 wird in den Einlaufbetrieb geschaltet, wobei gleichzeitig die Heizung für den Heizbeutel 72 aktiviert wird.

Die Steuereinrichtung 82 schaltet anschließend die ersten und zweiten Ventilpaare 46, 52 bzw. 50, 48 alternierend, sobald die erste bzw. zweite Bilanzkammerhälfte 32, 34 gefüllt ist. Durch die Füllung der Bilanzkammern 32 bzw. 34 erfolgt ein Druckanstieg in der Zuführungsleitung 20, 22, 24, der mit dem Senor 64 ermittelt und dann zur Auslösung des Umschaltsignals herangezogen wird.

Frische Dialysierflüssigkeit wird über die Leitungen 16, 20, 22 bzw. 24 der ersten Bilanzkammer 32 bzw. der zweiten Bilanzkammer 34 zugeführt. Die jeweils andere Bilanzkammerhälfte, die vollständig gefüllt ist, wird bei dem Füllen der komplementären Bilanzkammer durch Verdrängen der bewegbaren Membran 30 über die Leitung 36 bzw. 38 und die Katheterleitung 42 zum Patienten hin verdrängt. Dadurch wird eine im wesentlichen kontinuierliche Flüssigkeitsförderung zum Patienten erzeugt, die schneller und schonender ist.

Jedes Umschaltsignal entspricht also einem verdrängten Bilanzkammervolumenteil Dialysierflüssigkeit. Diese Umschaltsignale werden in der Steuereinheit 82 summiert und ergeben am Ende der Einlaufphase durch Multiplikation mit dem bekannten Bilanzkammervolumen die absolut zugeführte Dialysierflüssigkeitsmenge.

Mit dem ersten Drucksensor 56 wird dabei der Zuführungsdruck in der Katheterleitung 42 gemessen. In der Steuereinheit 82 ist ein vorgegebener Überdruck für die Zuführungsphase, beispielsweise +100 mbar festgelegt, der nicht überschritten werden darf. Demzufolge wird also die Pumprate an der Pumpe 60 so geregelt, daß dieser positive Grenzdruck nicht überschritten wird.

Hierdurch wird eine Befüllung in kürzestmöglicher, physiologisch verträglicher Zeit erreicht, ohne daß gefährliche Überdrucksituationen auftreten können.

Die Umschaltung der Bilanzkammerventile 46-52 kann nicht nur durch Anstieg des Drucks an dem zweiten Drucksensor 64 bestimmt werden, sie kann auch durch den Motorstromanstieg der Pumpe 60 ermittelt werden. Zweckmäßig ist es dabei, daß beim Umschalten alle Bilanzkammerventile 46-52 für eine kurze Zeitspanne geschlossen gehalten werden, so daß die Bilanz insgesamt nicht gestört wird. Der Vorgang der fortlaufenden Umschaltung führt dabei zu einem quasi-kontinuierlichen Fluß der Dialysierflüssigkeit durch die Bilanzkammer 26 und damit zum Peritonealkatheter.

Die Einlaufphase wird dann beendet, wenn die Steuereinheit 82 bei Erreichen einer vorgegebenen zugeführten Dialysierflüssigkeitsmenge (was durch die Zahl der Zyklen festgestellt werden kann) oder vorzugsweise nach Überschreiten eines vorbestimmten Überdrucks an dem ersten Drucksensor 56 ggf. bei einer immer geringer werdenden Förderraterate entsprechend aktiviert wird.

Am Ende der Einlaufphase wird das achte Ventil 78 geschlossen, so daß stromauf hiervon sämtliche übrigen Teile, Ventile und die Pumpe 60 desaktiviert werden können. Sofern überschüssige Dialysierflüssigkeit oder überschüssige Luft in der Katheterzuleitung vorhanden sein sollten, können diese Fluide über die Entlüftungsleitung 74 in den Ausguß entfernt werden, wobei hierzu das siebte Ventil 76 aktiviert wird. Diese Operation ist insbesondere dann wichtig, wenn ein Druckausgleich in der Katheterleitung 42 geschaffen werden soll, was üblicherweise vor Schließen des Ventils 78 geschieht.

Nach der Einlaufphase folgt die Behandlungsphase des Patienten, deren Dauer mit einem Zeitglied 83, das innerhalb der Steuereinheit 82 angeordnet ist, festgelegt ist.

Nach Ablauf dieses Peritonealdialysezyklus wird das Peritonealdialysegerät 10 in die Auslaufphase geschaltet. Hierzu wird zunächst das Ventil 78 geöffnet. Des gleichen werden die Ventilpaare 46 und 52 bzw. 48 und 50 wieder alternierend bei Erreichen des vollständigen Füllgrades der Bilanzkammer 26, was durch Überschreiten eines vorbestimmten Unterdrucks am zweiten Drucksensor 64 festgestellt wird, umgeschaltet. Die Abförderung der gebrauchten Dialysierflüssigkeit erfolgt mit der Pumpe 60, die durch das Steuergerät 82 in ihrem Drehsinn umgeschaltet wird (gegen den Uhrzeigersinn).

Nachdem sich die Pumpe 60 im Saugbetrieb befindet, stellt sich am Drucksensor 56 nunmehr ein Unterdruck ein, der bei Erreichen eines vorbestimmten Wertes zur Regelung der Pumprate herangezogen wird.

Des weiteren ist das Einlaufventil 68 geschlossen und das Auslaufventil 70 geöffnet. Das Entlüftungsventil 76 ist in dieser Phase geschlossen.

Die Abförderung der gebrauchten Dialysierflüssigkeit erfolgt unter Druckkontrolle mittels des ersten Drucksensors 56, wobei ein vorbestimmter Unterdruck, beispielsweise -100 mbar, nicht unterschritten werden darf.

Zunächst herrscht jedoch im Peritonealraum ein leichter Überdruck, so daß gebrauchte Dialysierflüssigkeit mit einer relativ hohen Entnahmerate entnommen werden kann. Am Ende der Auslaufrate steigt der Unterdruck an, so daß bei Erreichen des vorbestimmten Grenzwertes die Pumprate schrittweise zurückgenommen wird. Es kommt dann zu einer Zurückregelung der Fördermenge so lange, bis die Pumpe zum Stillstand kommt bzw. eine sehr geringe, vorgegebene Förderrate erreicht wird oder ein Bilanzkammerzyklus in dieser Phase abgeschlossen ist.

Dies ist ein Signal für das Ende der Entleerphase. Es werden dann wieder sämtliche Bilanzkammerhübe summiert und die gesamte Menge an entzogener Dialysierflüssigkeit bestimmt. Die Differenz zwischen zugeführter und abgeführter Flüssigkeitsmenge gibt dann die dem Patienten durch Ultrafiltration entzogene Flüssigkeitsmenge.

Am Ende dieser Phase wird die gesamte Anordnung gegenüber der Umgebung belüftet, wobei das Ventil 76 geöffnet wird. Sämtliche anderen Aggregate sind dabei desaktiviert. Anschließend wird das Ventil 78 geschlossen.

Danach erfolgt wiederum die Einleitung der Einlaufphase.

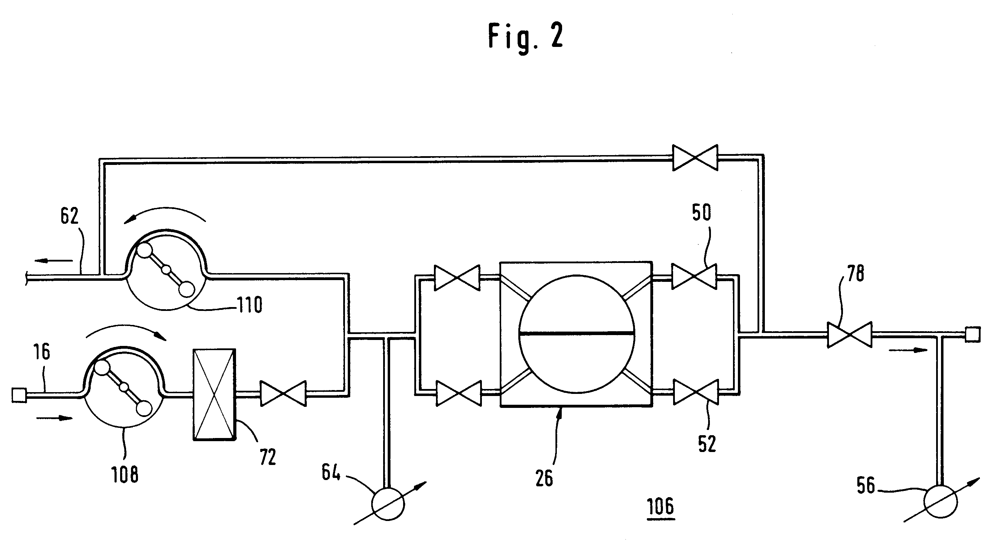

In Fig. 2 ist eine zweite Ausführungsform eines Peritonealdialysegerätes 106 gezeigt, die in ihrem Aufbau dem Peritonealdialysegerät 10 gemäß Fig. 1 weitgehend ähnlich ist. Insofern werden für gleiche Teile die gleichen Bezugszeichen verwandt.

Die zweite Ausführungsform unterscheidet sich gegenüber der ersten Ausführungsform lediglich dadurch, daß anstelle einer beiseitig betreibbaren Pumpe 60 zwei Pumpen eingesetzt werden. Dabei ist in der Zulaufleitung 16 stromauf des Ventils 68 eine Einlaufpumpe 108 als Peristaltikpumpe vorgesehen, während in der Auslaufleitung 62 eine Auslaufpumpe 110 vorgesehen ist, die ebenfalls als peristaltische Rollenpumpe ausgestaltet ist.

Hinzuzufügen ist, daß die Ventile 68 und 70 gemäß der zweiten Ausführungsform nicht notwendigerweise vorgesehen sein müssen, sofern die okkludierend wirkenden Peristaltikpumpen 108 und 110 eingesetzt werden..

Die Ausführungsform gemäß Fig. 2 wird in gleicher Weise wie die Ausführungsform gemäß Fig. 1 betrieben. Dabei wird in der Einlaufphase die Einlaufpumpe 108 aktiviert, während in der Auslaufphase nur die Auslaufpumpe 110 aktiviert ist. Die beiden Pumpen 108 und 110 wirken, wenn sie stillgesetzt sind, aufgrund ihrer okkludierenden Eigenschaften dabei als Schlauchklemmventile und sperren dabei die mit ihnen verbundenen Leitungen.

Sämtliche Ventile sind vorteilhafterweise als Klemmventile asgeführt und können teilweise auch in Gruppen ausgeführt oder geschaltet sein.

Zur Regelung und Überwachung der Heizung sind ein oder mehrere Temperatursensoren am Schlauch zweckmäßig. Auch kann ein Luftdetektor zwischen dem Ventil 78 und den Ventilen 50 und 52 zur Erhöhung der Sicherheit eingefügt sein.

Gemäß einer weiteren vorteilhaften Ausführungsform erfolgt eine redundante Drucküberwachung durch die beiden Drucksensoren 56 und 64, die hydraulisch durch die Leitungen und die Bilanzkammer 26 miteinander gekoppelt sind. Nach der Behandlung wird der Patient von der Kassette 80 abgetrennt, die danach verworfen wird.

## Patentansprüche

1. Peritonealdialysegerät (10,106) mit einer Bilanzieranordnung mit zwei Kammern (32,34), die durch eine Ventilanordnung (46-52) jeweils in eine Füll- bzw. Entleerungsphase umschaltbar sind, einer mit der Bilanzieranordnung über eine Zuführleitung (16,20,22,24) verbundene Peritonealdialysierflüssigkeitsquelle (12), eine mit einem Peritonealkatheter verbindbaren, von der Bilanzieranordnung abgehende Katheterleitung (42), eine von der Bilanzieranordnung abgehende Abflußleitung (22,24,20,62), einer Pumpanordnung (60,108,110) zur Förderung von frischer bzw. verbrauchter Peritonealdialysierflüssigkeit und einer Steuereinheit zum Steuern der Ventilanordnung (46-52) und der Pumpanordnung (60,108,110), **dadurch gekennzeichnet**, daß das Peritonealdialysegerät eine einzige Bilanzkammer (26) aufweist, die durch eine bewegliche, flüssigkeitsundurchlässige Membran (30) in zwei Bilanzkammerhälften (32, 34) getrennt sind, wobei die Bilanzkammerhälften (32,34) wechselweise über die Ventilanordnung (46-52) einerseits mit der Quelle für die Peritonealdialysierflüssigkeit (12) im Zuförderbetrieb und mit der Abflußleitung im Abförderbetrieb und andererseits mit der Katheterleitung (42) in Strömungsverbindung sind, ein erster Drucksensor (56) an der Katheterleitung (42) angeordnet ist, auf dessen druckabhängiges Signal die Steuereinheit (82) bei Erreichen eines vorgegebenen Druckwerts die Pumpe inaktiviert, und eine Einrichtung zum Ermitteln der vollständigen Füllung der Bilanzkammer vorgesehen ist, auf deren Signal hin die Steuereinheit (82) die Ventilanordnung jeweils zyklisch umschaltet.

2. Peritonealdialysegerät nach Anspruch 1, **dadurch gekennzeichnet**, daß die Steuereinheit (82) in der Entleerungsphase die Pumprate so regelt, daß ein vorbestimmter Unterdruck nicht unterschritten wird, und die Pumpanordnung (10,110) bei Erreichen einer vorbestimmten minimalen Pumprate, ggf. am Ende des Bilanzkammerhubes, der unmittelbar vor Erreichen dieser vorbestimmten Minimalpumprate liegt, abschaltet.

3. Peritonealdialysegerät nach Anspruch 1, **dadurch gekennzeichnet**, daß die Steuereinheit (82) den Füllbetrieb unter Abschalten der Pumpanordnung (60,108) und Umschalten der Bilanzieranordnung (46-52) beendet, wenn ein vorbestimmtes Volumen Peritonealdialysierflüssigkeit zugeführt worden ist.

4. Peritonealdialysegerät nach Anspruch 1, **dadurch gekennzeichnet**, daß die Steuereinheit (82) sowohl am Ende der Füllphase als auch am Ende der Entleerungsphase aus der Zahl der Umschaltzyklen der Ventilanordnung (46-52) und dem bekannten Innenvolumen der Bilanzkammer (26) das durch die Bilanzkammer (26) jeweils geförderte Volumen Peritonealdialysierflüssigkeit bestimmt und aus der Differenz von gebrauchtem und frischem Dialysierflüssigkeitsvolumen das jeweilige Ultrafiltrationsvolumen ermittelt.

5. Peritonealdialysegerät nach Anspruch 1, **gekennzeichnet durch** eine von der Dialysierflüssigkeitsquelle (12) abgehenden Einlaufleitung (14), die bis zu einem ersten Verzweigungspunkt (18) geführt ist, eine Abflußleitung (62), die vom ersten Verzweigungspunkt (18) abgeht und in einen Abfluß mündet, eine vom ersten Verzweigungspunkt (18) abgehende Förderleitung (20), deren Ende sich in eine erste Bilanzkammerleitung (22) und in eine zweite Bilanzkammerleitung (24) verzweigt, wobei die erste Bilanzkammerleitung (22) mit der ersten Bilanzkammerhälfte (32) und die zweite Bilanzkammerleitung (24) mit der zweiten Bilanzkammerhälfte (34) in Strömungsverbindung sind,
eine von der ersten Bilanzkammerhälfte (32) abgehenden dritten Bilanzkammerleitung (36) und eine von der zweiten Bilanzkammerhälfte (34) abgehenden vierten Bilanzkammerleitung (38), die jeweils in einem zweiten Verbindungspunkt (40) münden, von dem eine Katheterleitung (42) abgeht, deren Ende mit einem Peritonealkatheter verbindbar ist, und einem ersten, zweiten, dritten bzw. vierten Ventil (46-52), die in die erste, zweite, dritte bzw. vierte Bilanzkammerleitung (22,24,36,38) eingeschaltet sind.

6. Peritonealdialysegerät nach Anspruch 1**, dadurch gekennzeichnet**, daß in der Förderleitung (20) eine beidseitig betreibbare peristaltische Pumpe (60) angeordnet ist und die Einlaufleitung (16) ein siebtes Ventil (68), das in der Einlaufphase geöffnet ist, und die Abflußleitung (62) ein achtes Ventil, das in der Abflußphase geöffnet ist, aufweisen.

7. Peritonealdialysegerät nach Anspruch 1, **dadurch gekennzeichnet**, daß in der Zuführleitung (16) eine Zuführpumpe (108) und in der Abflußleitung (62) eine Abflußpumpe (110) angeordnet sind.

8. Peritonealdialysegerät nach Anspruch 1, **dadurch gekennzeichnet**, daß die Einrichtung zur Ermittlung der vollständigen Füllung der Bilanzkammer (26) ein zweiter Drucksensor (64) ist, der mit der Förderleitung (20) verbunden ist.

9. Peritonealdialysegerät nach Anspruch 1, **dadurch gekennzeichnet**, daß in der Katheterleitung (42) ein fünftes Ventil (78) angeordnet ist.

10. Peritonealdialysegerät nach Anspruch 9, **dadurch gekennzeichnet**, daß zwischen dem zweiten Verbindungspunkt (40) und dem fünften Ventil (78) eine Bypass-Leitung (74) von der Katheterleitung (52) abgeht, in die zur Belüftung der Katheterleitung (42) ein sechstes Ventil (76) eingeschaltet ist und deren Ende in die Abflußleitung (62) mündet.

11. Einmalschlauchsystem für das Peritonealdialysegerät nach Anspruch 1, aufweisend eine Bilanzkammer (26), die durch eine bewegliche, flüssigkeitsundurchlässige Membran (30) in zwei Bilanzkammerhälften (32) und (34) geteilt ist, eine erste Bilanzkammerleitung (22), die von der ersten Bilanzkammerhälfte (32) abgeht, und eine zweite Bilanzkammerleitung (24), die von der zweiten Bilanzkammerhälfte (34) abgeht, eine Förderleitung (20), deren erstes Ende mit den Enden der ersten Bilanzkammerleitung (22) und der zweiten Bilanzkammerleitung (24) vereinigt ist und deren zweites Ende sich in eine Zuführleitung (16), die geeignet ist, mit einer Quelle (12) für Peritonealdialysierflüssigkeit verbunden zu werden, und eine Abführleitung (62) verzweigt, einer dritten Bilanzkammerleitung (36), die von der ersten Bilanzkammerhälfte (32) abgeht, und einer vierten Bilanzkammerleitung (38), die von der zweiten Bilanzkammerhälfte (34) abgeht, und einer Katheterleitung (42), deren erstes Ende mit den Enden der Bilanzkammerleitungen (36) und (38) verbunden ist und deren zweites Ende geeignet ist, mit einem Peritonealkatheter verbunden zu werden.

12. System nach Anspruch 11, **dadurch gekennzeichnet**, daß von der Katheterleitung (42) eine Bypassleitung (74) abzweigt, die mit der Abflußleitung (62) verbunden ist.
